# EUROPÄISCHE PATENTSCHRIFT

(11) **EP 0 613 366 B1**
(45) Veröffentlichungstag und Bekanntmachung des Hinweises auf die Patenterteilung: **20.12.1995**
(21) Anmeldenummer: 92923384.9
(22) Anmeldetag: 10.11.1992
(51) Int. Cl.: A61K 7/13

(54) **5,6-DIHYDROXYINDOLINE ALS ADDITIV ZU HAARFÄRBEREZEPTUREN**
5,6-DIHYDROXYINDOLINE AS ADDITIVES FOR HAIR-DYEING PREPARATIONS
5,6-DIHYDROXYINDOLINES UTILISEES COMME ADJUVANTS A DES FORMULATIONS DE TEINTURES POUR CHEVEUX

(30) Priorität: 19.11.1991 DE 4137971
(43) Veröffentlichungstag der Anmeldung: 07.09.1994
(73) Patentinhaber: Henkel Kommanditgesellschaft auf Aktien, 40191 Düsseldorf (DE)
(72) Erfinder: MATZIK, Iduna, D-4006 Erkrath 2 (DE); HOLLENBERG, Detlef, D-4006 Erkrath 2 (DE); BORGERDING, Mechthild, D-4018 Langenfeld (DE)
(86) Internationale Anmeldenummer: EP9202578
(87) Internationale Veröffentlichungsnummer: WO9309759

(56) Entgegenhaltungen:
- WO-A-91/17739
- DE-A- 1 916 139
- US-A- 4 013 404

## Beschreibung

Die Erfindung betrifft Haarfärbemittel auf der Basis von direktziehenden Farbstoffen oder von Oxidationsfarbstoffvorprodukten des Entwickler- und Kuppler-Typs, die bestimmte Indoline, insbesondere 5,6-Dihydroxyindoline, enthalten.

Die farbgebenden Komponenten in herkömmlichen Haarfärberezepturen sind meist entweder direktziehende Farbstoffe oder Farbstoffe, die sich aus Oxidationsfarbstoffvorprodukten vom Entwickler- und Kuppler-Typ durch Oxidation mit Luftsauerstoff oder mit anderen Oxidationsmitteln (z. B. H₂O₂) bilden. Zur Verwendung in Haarfärbemitteln werden diese in einen kosmetischen Träger eingearbeitet. Als direktziehende Farbstoffe werden z. B. Nitrophenylendiaminderivate, Anthrachinonderivate oder Naphtochinonderivate, eingesetzt.

Als Oxidationsfarbstoffe verwendet man üblicherweise eine Kombination aus einer Entwicklerkomponente, z. B. einem primären aromatischen Amin mit einer weiteren in para- oder orthoposition befindlichen freien oder substituierten Hydroxy- oder Aminogruppe, Diaminopyridine, heterocyclische Hydrazonderivate, 4-Aminopyrazolonderivate oder Tetraaminopyridine und einer Kupplerkomponente, z. B. m-Phenylendiamine, 3-Aminophenole, Resorcine, Naphtole, Pyrazolone. Diese reagieren in Gegenwart von Luftsauerstoff oder anderen Oxidationsmitteln unter Kupplung zu den eigentlichen Farbstoffen.

Gute Haarfärbemittel müssen in erster Linie folgende Voraussetzungen erfüllen: Sie müssen die gewünschte Farbnuance in ausreichender Intensität ausbilden. Sie müssen ferner ein gutes Aufziehvermögen auf menschlichem Haar besitzen, ohne die Kopfhaut zu stark anzufärben. Die erzeugten Färbungen müssen eine hohe Stabilität gegen Wärme, Licht, Haarwaschmittel und die bei der Dauerwellung verwendeten Chemikalien aufweisen. Schließlich sollen Haarfärbemittel in toxikologischer und dermatologischer Hinsicht unbedenklich sein.

Es besteht daher ein stetes Bedürfnis nach Haarfärberezepturen mit verbesserten toxikologischen und anwendungstechnischen Eigenschaften, insbesondere hinsichtlich der Echtheiten der erzielten Haarfärbungen.

Es wurde nunmehr eine überraschende Möglichkeit gefunden, die Färbeeigenschaften von herkömmlichen Haarfärberezepturen sowohl vom Entwickler- und Kuppler-Typ als auch auf der Basis von direktziehenden Farbstoffen zu verbessern.

Durch die Verwendung nur geringer Mengen von Indolinen der Formel I 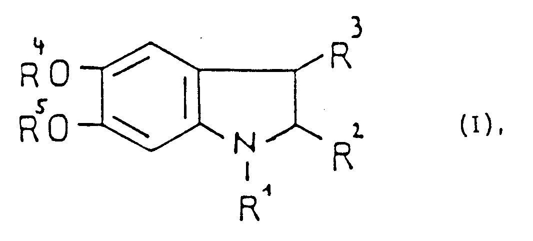
worin R¹, R², R³, R⁴ und R⁵ unabhängig voneinander Wasserstoff oder Alkylgruppen mit 1 bis 4 C-Atomen oder R⁴ und R⁵ gemeinsam mit den Sauerstoffatomen, an die sie gebunden sind, eine Alkylendioxygruppe mit 1 bis 4 C-Atomen darstellen, oder deren Salzen als Additiv in Haarfärberezepturen mit üblichen direktziehenden Farbstoffen oder in Haarfärberezepturen mit Oxidationsfarbstoffvorprodukten vom Entwickler- und Kuppler-Typ lassen sich Haarfärbungen erzielen, die die mit bekannten Haarfärbemitteln erzielten Nuancen hinsichtlich der Intensität und Echtheiten deutlich übertreffen.

Wegen der chemischen Analogie der Indoline der Formel I zu den Grundbausteinen des im Haar enthaltenen natürlichen Melanin-Farbstoffs kann auch ein günstiges toxikologisches und dermatologisches Verhalten der Indoline enthaltenden Haarfärbemittel erwartet werden.

Das 5,6-Dimethoxyindolin und das 5,6-Dihydroxyindolin sind literaturbekannt, ihre Herstellung ist z.B. in J. Chem. Soc. (C), 1967, Seiten 1424 bis 1427 beschrieben. In analoger Weise lassen sich die alkylsubstituierten Indoline der Formel I aus den entsprechend substituierten 5,6-Dihydroxyindolen oder Alkoxyindolen durch katalytische Hydrierung herstellen. Ein anderes Verfahren zur Herstellung von 5,6-Dihydroxyindolinen aus 5,6-Dimethoxyindolen durch Reduktion mit Natrium-cyanoborhydrid und Abspaltung der Methoxygruppen in konzentrierter Salzsäure ist im Journal of Medicinal Chemistry, 1978, Vol 21, No. 6, Seite 553 beschrieben.

Bevorzugt geeignet sind Indoline der Formel I, bei welchen eine der Gruppen R¹, R² und R³ eine Methylgruppe ist und die übrigen Wasserstoffe sind, ganz besonders jedoch eignet sich der Grundkörper 5,6-Dihydroxyindolin.

Die Indoline der Formel I oder deren Salze werden einerseits Haarfärbemittelrezepturen zugesetzt, die Oxidationsfarbstoffvorprodukte vom Entwickler- und Kuppler-Typ enthalten, wobei ein 3-Komponentensystem zusammengesetzt aus üblichem Entwickler, üblichem Kuppler und dem Indolinderivat entsteht; es ist jedoch nicht erforderlich, daß nur ein Kuppler und ein Entwickler verwendet wird, vielmehr können auch Gemische von üblichen Kupplern und Gemische von üblichen Entwicklern anstelle der Einzelkomponenten zum Einsatz kommen. Andererseits werden die Indoline oder deren Salze Haarfärbemittelrezepturen zugesetzt, die übliche direktziehende Farbstoffe enthalten.

Die Indoline der Formel I können dabei in freier Form oder in Form ihrer Salze, bevorzugt als Hydrochloride, Hydrobromide, Sulfate, Phosphate, Acetate, Propionate, Lactate oder Citrate eingesetzt werden.

Gegenstand der vorliegenden Erfindung sind Haarfärbemittel, enthaltend Indoline der Formel I 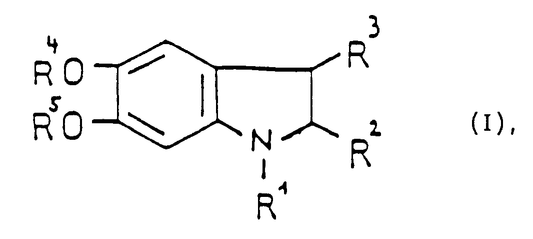
worin R¹, R², R³, R⁴ und R⁵ unabhängig voneinander Wasserstoffe oder Alkylgruppen mit 1 bis 4 C-Atomen oder R⁴ und R⁵ gemeinsam mit den Sauerstoffatomen, an die sie gebunden sind, eine Alkylendioxygruppe mit 1 bis 4 C-Atomen darstellen, oder deren Salze in einem Träger, dadurch gekennzeichnet, daß neben den. Indolinen der Formel I oder deren Salzen in einer Menge von 0,05 bis 5 Gew.-% des gesamten Haarfärbemittels übliche direktziehende Farbstoffe in einer Menge von 0,05 bis 5 Gew.-% des gesamten Haarfärbemittels enthalten sind.

Als übliche direktziehende Farbstoffe können alle hierfür bekannten Verbindungen eingesetzt werden; z. B. 2-Nitro-p-phenylendiamin, 6-Chlor-4-nitro-2-aminophenol, 2-Amino-4,6-dinitrophenol, 1-(β-Hydroxyethyl)amino-2-nitro-4-bis(β-hydroxyethyl)aminobenzol, 1-(β-Hydroxyethyl)-amino-2-nitro-4-aminobenzol, 1-(β-Hydroxyethyl)amino-2-nitrobenzol 1,4-Diamino-5-nitro-anthrachinon, 1,4-Diaminoanthrachinon, 1,4,5,8-Tetraaminoanthrachinon, 1-Amino-4-methylaminoanthrachinon.

Ein weiterer Erfindungsgegenstand sind Haarfärbemittel, enthaltend Indoline der Formel I 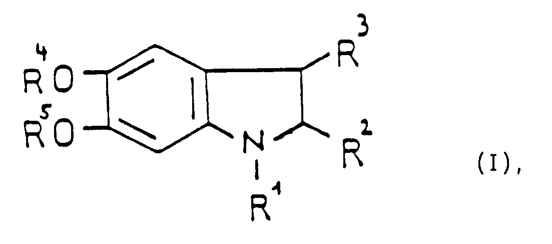
worin R¹, R², R³, R⁴ und R⁵ unabhängig voneinander Wasserstoffe oder Alkylgruppen mit 1 bis 4 C-Atomen oder R⁴ und R⁵ gemeinsam mit den Sauerstoffatomen, an die sie gebunden sind, eine Alkylendioxygruppe mit 1 bis 4 C-Atomen darstellen, oder deren Salze in einem Träger, dadurch gekennzeichnet, daß neben den. Indolinen der Formel I oder deren Salzen in einer Menge von 0,05 bis 5 Gew.-% des gesamten Haarfärbemittels übliche Entwicklerverbindungen in einer Menge von 0,05 bis 5 Gew.-% des gesamten Haarfärbemittels und übliche Kupplerverbindungen in einer Menge von 0,05 bis 5 Gew.-% des gesamten Haarfärbemittels enthalten sind.

Als übliche Kuppler und Entwickler können alle hierfür bekannten Verbindungen eingesetzt werden, als Kuppler beispielsweise Resorcin, 2-Methylresorcin, α-Naphtol, 1,5-Dihydroxynaphtalin, 3-Aminophenol, p-Amino-o-cresol, 2-Chlor-3-amino-6-methylphenol, 2,4-Dichlor-3-aminophenol, 1,3-Bis-(2,4-diaminophenoxy)-propan, 1-Phenyl-3-methyl-pyrazlon-5, 1-Phenyl-3-aminopyrazolon-5, und als Entwickler z. B. p-Phenylendiamin, p-Toluylendiamin, 2-Chlor-p-phenyldiamin, N-Methyl-p-phenylendiamin, p-Aminophenol.

In den Oxidationshaarfarbstoffen sind die Entwickler bzw. Kuppler jeweils in Mengen vom 0,05 bis 5 Gew.-% vorzugsweise 0,1 bis 2 Gew.-% des gesamten Haarfärbemittels enthalten. In den auf direktziehenden Farbstoffen basierenden Rezepturen sind direktziehende Farbstoffe in einer Menge von 0,05 bis 5 Gew.-%, vorzugsweise 0,1 bis 2 Gew.-% des gesamten Haarfärbemittels enthalten. Die Indoline der Formel I werden jeweils in Mengen von 0,05 bis 5 Gew.-% vorzugsweise 0,05 bis 2 Gew.-% des gesamten Haarfärbemittel zugesetzt.

Die Indoline der Formel I modifizieren dabei die mit den obengenannten herkömmlichen Haarfärbungssystemen erzielten Färbungen dahingehend, daß die Farbtiefe und die Echtheiten deutlich verbessert werden.

Zur Herstellung der erfindungsgemäßen Haarfärbemittel werden die direktziehenden Farbstoffe bzw. die Oxidationsfarbstoffvorprodukte vom Kuppler- und Entwickler-Typ in einen geeigneten kosmetischen Träger eingearbeitet. Solche Träger sind z. B. Cremes, Emulsionen, Gele oder auch tensidhaltige, schäumende Lösungen, z. B. Shampoos, Schaumaerosole oder andere Zubereitungen, die für die Anwendung auf dem Haar geeignet sind.

Übliche Bestandteile solcher kosmetischer Zubereitungen sind z. B.
- Netz- und Emulgiermittel wie anionische, nichtionische oder ampholytische Tenside, z. B. Fettalkoholsulfate, Alkansulfonate, α-Olefinsulfonate, Fettalkoholpolyglcolethersulfate, Ethylenoxidanlagerungsprodukte an Fettalkohole, an Fettsäuren, an Alkylphenole, an Sorbitanfettsäureester, an Fettsäurepartialglyceride und an Fettsäurealkanolamide,
- Verdickungsmittel, wie z. B. Fettalkohole, Fettsäuren, Paraffinöle, Fettsäureester und andere Fettkomponenten in emulgierter Form,
- wasserlösliche, polymere Verdickungsmittel, wie z. B. Methyl- oder Hydroxyethylcellulose, Stärke, Pflanzengumme, wasserlösliche synthetische Polymerisate, wasserlösliche Biopolymere (z. B. Xanthan-Gum),
- haarpflegende Zusätze, wie z. B. wasserlösliche, kationische Polymere, Proteinderivate, Pantothensäure, Vitamine, Pflanzenextrakte, Cholesterin und Zucker,
- Elektrolyt- und Puffersalze, pH-Stellmittel, Komplexbildner und Parfümöle,
- Reduktionsmittel zur Stabilisierung des Farbstoffs, z. B. Natriumsulfit oder Ascorbinsäure.

Die Bestandteile der kosmetischen Träger werden zur Herstellung der erfindungsgemäßen Haarfärbemittel in für diesen Zweck üblichen Mengen eingesetzt; z. B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Besonders geeignet als Träger ist eine Öl-in-Wasser-Emulsion mit einem Gehalt von 1 bis 25 Gew.-% einer Fettkomponente und 0,5 bis 30 Gew.-% eines Emulgierungsmittels aus der Gruppe der anionischen, nichtionischen, ampholytischen oder zwitterionischen Tenside. Falls die erfindungsgemäß zu verwendenden Indoline als Additiv zu Haarfärbemitteln vom Entwickler- und Kuppler-Typ eingesetzt Werden, so werden Entwickler und Kuppler jeweils in Mengen von 0,05 bis 5 Gew.-%, vorzugsweise 0,1 bis 2 Gew.-%, des gesamten Färbemittels, die Indoline der Formel I in Mengen von 0,05 bis 5 Gew.-%, vorzugsweise 0,05 bis 2 Gew.-% des gesamten Färbemittels in den Träger eingemischt.

Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein Aufhelleffekt am Haar gewünscht wird. Als Oxidationsmittel kommen insbesondere Wasserstoffperoxid oder dessen Anlagerungsprodukte an Harnstoff, Melamin oder Natriumborat sowie Gemische aus derartigen Wasserstoffperoxidanlagerungsprodukten mit Kaliumperoxiddisulfat in Betracht.

Die Anwendung der erfindungsgemäßen Haarfärbemittel kann, unabhängig von der Art der kosmetischen Zubereitung, zum Beispiel als Creme, Gel oder Shampoo, im schwach sauren, neutralen oder alkalischen Milieu erfolgen. Bevorzugt ist die Anwendung der Haarfärbemittel in einem pH-Bereich von 6 bis 10. Die Anwendungstemperaturen können in einem Bereich zwischen 15°C und 40°C liegen. Nach einer Einwirkungszeit von ca. 30 Minuten wird das Haarfärbemittel durch Ausspülen von dem zu färbenden Haar entfernt. Danach wird das Haar mit einem milden Shampoo nachgewaschen und getrocknet. Das Nachwaschen mit einem Shampoo entfällt, wenn ein stark tensidhaltiger Träger, z. B. ein Färbeshampoo, verwendet wurde.

Die nachfolgenden Beispiele sollen den Erfindungsgegenstand näher erläutern, ohne ihn jedoch hierauf zu beschränken.

### Beispiele

### Anwendungsbeispiele

### 5,6-Dihydroxyindolin als Additiv zu Haarfärbemitteln vom Kuppler- und Entwickler-Typ:

### Beispiel 1

| | Vergleich | Erfindungsgemäß |
|---|---|---|
| Talgfettalkohol | 6,5 g | 6,5 g |
| Kokoslorol | 2,0 g | 2,0 g |
| Laurylethersulfat (Texapon^{(R)} N 25) | 7,28 g | 7,28 g |
| p-Aminophenol · HCl | 0,44 g | 0,44 g |
| p-Toluylendiamin · HCl | 0,31 g | 0,31 g |
| m-Aminophenol | 0,114 g | 0,114 g |
| p-Amino-o-cresol | 0,11 g | 0,11 g |
| 2-Methylresorcin | 0,08 g | 0,08 g |
| 5,6-Dihydroxyindolin | - | 0,5 g |
| Na₂SO₃ | 0,5 g | 0,5 g |
| (NH₄)₂SO₄ | 0,8 g | 0,8 g |
| Konz. NH₃-Lösung | bis pH=9,5 | bis pH=9,5 |
| Wasser | ad 100 g | ad 100 g |

Die Bestandteile wurden der Reihe nach miteinander vermischt. Nach Zugabe der Oxidationsfärbemittelvorprodukte und des Inhibitors wurde zunächst mit konzentrierter Ammoniak-Lösung der pH-Wert der Emulsion auf 9,5 eingestellt, dann wurde mit Wasser auf 100 g aufgefüllt.

Die oxidative Entwicklung der Färbung wurde mit 3 %iger Wasserstoffperoxidlösung als Oxidationsmittel durchgeführt. Hierzu wurden 100 g der Emulsion mit 50 g Wasserstoffperoxidlösung (3 %ig) versetzt und vermischt.

Die Färbecreme wurde auf ca. 5 cm lange Strähnen standardisierten, zu 90 % ergrauten, aber nicht besonders vorbehandelten Menschenhaars aufgetragen und dort 30 Minuten bei 27 °C belassen. Nach Beendigung des Färbeprozesses wurde das Haar gespült, mit einem üblichen Haarwaschmittel ausgewaschen und anschließend getrocknet.

Bei Verwendung des erfindungsgemäßen Haarfärbemittels 1 resultierte eine rotbraune Färbung mit sehr guter Grauabdeckung und sehr guter Waschechtheit. Nach 6 Wäschen mit einem handelsüblichen Haarshampoo verblieb eine Restfärbung von 95 % gegenüber 62 % Restfärbung bei Verwendung des Vergleichs-Haarfärbemittels 1.

### Beispiel 2

| | Vergleich | Erfindungsgemäß |
|---|---|---|
| Talgfettalkohol | 6,5 g | 6,5 g |
| Kokoslorol | 2,0 g | 2,0 g |
| Laurylethersulfat (Texapon N 25) | 7,28 g | 7,28 g |
| p-Aminophenol | 0,3 g | 0,3 g |
| p-Toluylendiamin | 0,02 g | 0,02 g |
| p-Amino-o-cresol | 0,05 g | 0,05 g |
| 5,6-Dihydroxyindolin | - | 0,3 g |
| Na₂SO₃ | 0,5 g | 0,5 g |
| (NH₄)₂ SO₄ | 1,0 g | 1,0 g |
| Konz. NH₃-Lsg. | bis pH=9,5 | bis pH=9,5 |
| Wasser | ad 100 g | ad 100 g |

Die Anwendungen erfolgten wie im Beispiel 1.

Bei Verwendung des erfindungsgemäßen Haarfärbemittels 2 resultierte eine goldblonde Färbung mit sehr guter Waschechtheit. Nach 6 Wäschen verblieb eine Restfärbung von 92 % gegenüber 65 % Restfärbung bei Verwendung des Vergleichs-Haarfärbemittels 2.

### Beispiel 3

| | Vergleich | Erfindungsgemäß |
|---|---|---|
| Talgfettalkohol | 6,5 g | 6,5 g |
| Kokoslorol | 2,0 g | 2,0 g |
| Laurylethersulfat (Texapon N 25) | 7,28 g | 7,28 g |
| p-Aminophenol | 0,14 g | 0,14 g |
| p-Toluylendiamin | 1,10 g | 1,10 g |
| Resorcin | 0,08 g | 0,08 g |
| 2-Methylresorcin | 0,105 g | 0,105 g |
| 2,4-Dichlor-3-aminophenol · HCl | 0,04 g | 0,04 g |
| 5,6-Dihydroxyindolin | - | 0,5 g |
| Na₂SO₃ | 0,5 g | 0,5 g |
| (NH₄)₂ SO₄ | 0,8 g | 0,8 g |
| Konz. NH₃-Lsg. | bis pH=9,5 | bis pH=9,5 |
| Wasser | ad 100 g | ad 100 g |

Die Anwendungen erfolgten wie in den Beispielen 2 und 3.

Bei Verwendung des erfindungsgemäßen Haarfärbemittels 3 resultierte eine dunkelbraune Färbung mit vollständiger Grauabdeckung und sehr guter Waschechtheit. Nach 6 Wäschen verblieb eine Restfärbung von 96 % gegenüber 78 % Restfärbung bei Verwendung des Vergleichs-Haarfärbemittels 3.

5,6-Dihydroxyindolin als Additiv zu Haarfärmitteln auf der Basis von direktziehenden Farbstoffen.

### Beispiel 4

| | Vergleich | Erfindungsgemäß |
|---|---|---|
| Talgfettalkohol | 4,0 g | 4,0 g |
| Kokoslorol | 1,0 g | 1,0 g |
| Eumulgin B1 | 2,0 g | 2,0 g |
| 1-(β-Hydroxyethyl)amino-2-nitro-4-bis(β-hydroxyethyl)aminobenzol | 1,0 g | 1,0 g |
| 1-(β-Hydroxyethyl)amino-2-nitro-4-aminobenzol | 0,1 g | 0,1 g |
| 1-(β-Hydroxyethyl)amino-2-nitrobenzol | 0,2 g | 0,2 g |
| 5,6-Dihydroxyindolin | - | 0,2 g |
| Wasser | ad 100 g | ad 100 g |

Vergleichs-Haarfärbemittel bzw. erfindungsgemäßes Haarfärbemittel wurden auf 5 cm lange Strähnen, standardisierten, zu 90 % ergrautem, aber nicht besonders vorbehandelten Menschenhaars aufgetragen und dort ca. 20 Minuten bei 27 °C belassen, dann mit einem üblichen Haarwaschmittel ausgewaschen, mit Wasser gespült und getrocknet. Bei Verwendung des erfindungsgemäßen Haarfärbemittels 4 resultierte eine mittelbraune Färbung mit guter Grauabdeckung und guter Waschechtheit. Nach 6 Wäschen verblieb eine Restfärbung von 75 % gegenüber 40 % Restfärbung bei Verwendung des Vergleichs-Haarfärbemittels 4.

## Patentansprüche

1. Haarfärbemittel, enthaltend Indoline der Formel I 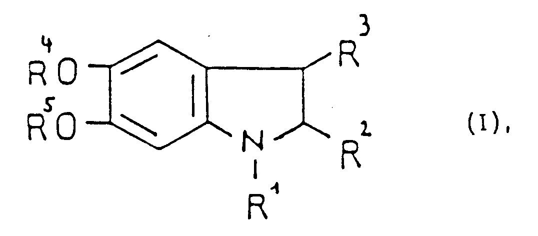 worin R¹, R², R³, R⁴ und R⁵ unabhängig voneinander Wasserstoffe oder Alkylgruppen mit 1 bis 4 C-Atomen oder R⁴ und R⁵ gemeinsam mit den Sauerstoffatomen, an die sie gebunden sind, eine Alkylendioxygruppe mit 1 bis 4 C-Atomen darstellen, oder deren Salze in einem Träger, dadurch gekennzeichnet, daß neben den Indolinen der Formel I oder deren Salzen in einer Menge von 0,05 bis 5 Gew.-% des gesamten Haarfärbemittels übliche direktziehende Farbstoffe in einer Menge von 0,05 bis 5 Gew.-% des gesamten Haarfärbemittels enthalten sind.

2. Haarfärbemittel, enthaltend Indoline der Formel I 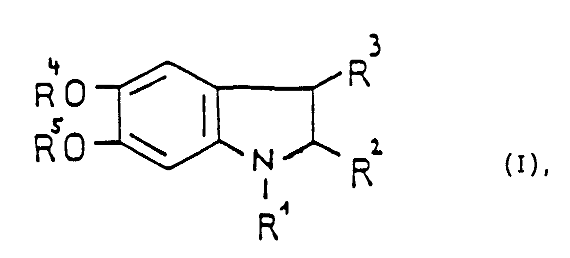 worin R¹, R², R³, R⁴ und R⁵ unabhängig voneinander Wasserstoffe oder Alkylgruppen mit 1 bis 4 C-Atomen oder R⁴ und R⁵ gemeinsam mit den Sauerstoffatomen, an die sie gebunden sind, eine Alkylendioxygruppe mit 1 bis 4 C-Atomen darstellen, oder deren Salze in einem Träger, dadurch gekennzeichnet, daß neben den Indolinen der Formel I oder deren Salzen in einer Menge von 0,05 bis 5 Gew.-% des gesamten Haarfärbemittels übliche Entwicklerverbindungen in einer Menge von 0,05 bis 5 Gew.-% des gesamten Haarfärbemittels und übliche Kupplerverbindungen in einer Menge von 0,05 bis 5 Gew.-% des gesamten Haarfärbemittels enthalten sind.

3. Haarfärbemittel nach einem der Ansprüche 1 und 2, dadurch gekennzeichnet, daß in der Formel I die Gruppen R¹, R², R³, R⁴ und R⁵ Wasserstoffe darstellen, und gegebenenfalls eine der Gruppen R¹, R² und R³ eine Methylgruppe ist.

## Claims

1. Hair colouring formulations containing indolines corresponding to formula I: 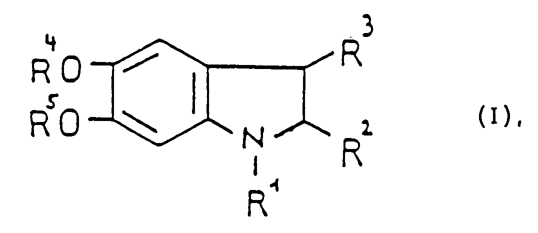 in which R¹, R², R³, R⁴ and R⁵ independently of one another represent hydrogens or C₁₋₄ alkyl groups or R⁴ and R⁵ together with the oxygen atoms to which they are attached represent a C₁₋₄ alkylenedioxy group,
or salts thereof in a carrier, characterized in that, in addition to the indolines corresponding to formula I or salts thereof in a quantity of 0.05 to 5% by weight, based on the hair colouring formulation as a whole, they contain typical substantive dyes in a quantity of 0.05 to 5% by weight, based on the hair colouring formulation as a whole.

2. Hair colouring formulations containing indolines corresponding to formula I: 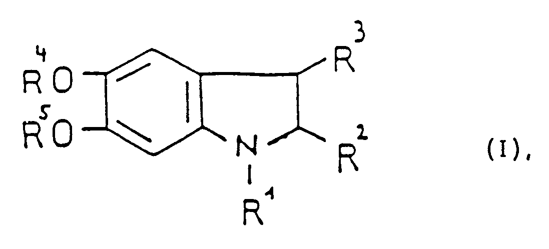 in which R¹, R², R³, R⁴ and R⁵ independently of one another represent hydrogens or C₁₋₄ alkyl groups or R⁴ and R⁵ together with the oxygen atoms to which they are attached represent a C₁₋₄ alkylenedioxy group, or salts thereof in a carrier
characterized in that, in addition to the indolines corresponding to formula I or salts thereof in a quantity of 0.05 to 5% by weight, based on the hair colouring formulation as a whole, they contain typical primary intermediates in a quantity of 0.05 to 5% by weight, based on the hair colouring formulation as a whole, and typical secondary intermediates in a quantity of 0.05 to 5% by weight, based on the hair colouring formulation as a whole.

3. Hair colouring formulations as claimed in any of claims 1 and 2, characterized in that the groups R¹, R², R³, R⁴ and R⁵ in formula I represent hydrogens and one of the groups R¹, R² and R³ is optionally a methyl group

## Revendications

1. Teinture pour cheveux contenant des indolines de formule I: 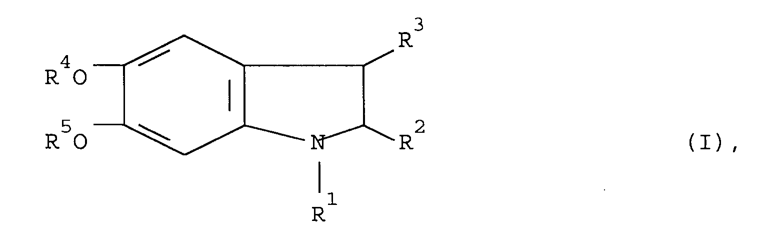 dans laquelle R¹, R², R³, R⁴ et R⁵ indépendamment l'un de l'autre représentent de l'hydrogène ou des groupes alcoyle ayant de 1 à 4 atomes de carbone ou bien R⁴ et R⁵ ensemble avec les atomes d'oxygène auxquels ils sont liés, représentent un groupe alcoylènedioxy ayant de 1 à 4 atomes de carbone, ou leurs sels, dans un support, caractérisée en ce qu'à côté des indolines de formule I ou de leurs sels en quantité allant de 0,05 à 5 % en poids de la teinture pour cheveux totale, des colorants usuels qui montent directement sont contenus en quantités allant de 0,05 à 5 % en poids de la teinture pour cheveux totale.

2. Teinture pour cheveux contenant des indolines de formule I : 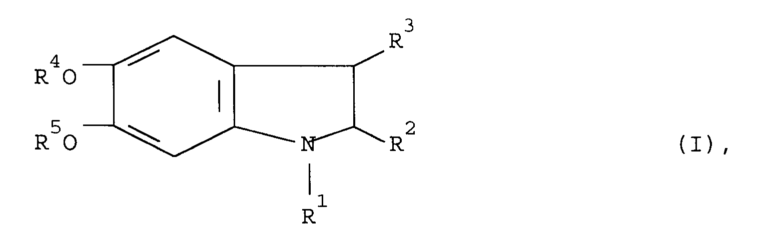 dans laquelle R¹, R², R³, R⁴ et R⁵ indépendamment l'un de l'autre représentent de l'hydrogène ou des groupes alcoyle ayant de 1 à 4 atomes de carbone ou bien R⁴ et R⁵ ensemble avec les atomes d'oxygène auxquels ils sont liés, représentent un groupe alcoylènedioxy ayant de 1 à 4 atomes de carbone, ou leurs sels, dans un support, caractérisée en ce qu'à côté des indolines de formule I ou de leurs sels, en quantités allant de 0,05 à 5 % en poids de la teinture pour cheveux totale, des composés révélateurs usuels sont contenus en quantités allant de 0,05 à 5 % en poids de la teinture pour cheveux, totale et des composés de couplage usuels en quantités allant de 0,05 à 5 % en poids de la teinture pour cheveux, totale.

3. Teinture pour cheveux selon l'une des revendications 1 et 2, caractérisée en ce que dans la formule I, les groupes R¹, R², R³, R⁴ et R⁵ représentent de l'hydrogène et le cas échéant un des groupes R¹, R² et R³ est un groupe méthyle.
